# FASCICULE DE BREVET EUROPEEN

(11) **EP 2 588 148 B1**
(45) Date de publication et mention de la délivrance du brevet: **10.12.2014**
(21) Numéro de dépôt: 11728007.3
(22) Date de dépôt: 29.06.2011
(51) Int. Cl.: A01N 25/04, A61L 2/23

(54) **GEL DE DÉCONTAMINATION BIOLOGIQUE ET PROCÉDÉ DE DÉCONTAMINATION DE SURFACES UTILISANT CE GEL**
BIOLOGISCHES DEKONTAMINATIONSGEL UND VERFAHREN ZUR DEKONTAMINATION VON OBERFLÄCHEN MIT DIESEM GEL
BIOLOGICAL DECONTAMINATION GEL, AND METHOD FOR DECONTAMINATING SURFACES USING SAID GEL

(30) Priorité: 02.07.2010 FR 1055399
(43) Date de publication de la demande: 08.05.2013
(73) Titulaire: Commissariat à l'Énergie Atomique et aux Énergies Alternatives, 75015 Paris (FR)
(72) Inventeur: CUER, Frédéric, F-30630 Cornillon (FR); FAURE, Sylvain, F-84120 Venasque (FR)
(74) Mandataire: Ilgart, Jean-Christophe
(86) Numéro de dépôt international: PCT/EP2011/060914
(87) Numéro de publication internationale: WO 2012/001046

(56) Documents cités:
- WO-A1-01/85845
- WO-A1-03/008529
- WO-A2-2007/039598
- US-B1- 6 455 751

## Description

### DOMAINE TECHNIQUE

La présente invention a pour objet un gel de décontamination biologique utilisable pour la décontamination de surfaces.

La présente invention a trait, en outre, à un procédé de décontamination de surfaces utilisant ce gel.

L'invention s'applique à la décontamination de surfaces polluées, contaminées, par des agents biologiques.

Le procédé selon l'invention peut s'appliquer à toutes sortes de surfaces telles que les surfaces métalliques, les surfaces en matières plastiques, les surfaces en matériaux vitreux.

L'invention s'applique tout particulièrement aux surfaces de matériaux poreux tels que les matrices, matériaux, cimentaires comme les pâtes, les mortiers et les bétons ; les briques ; les plâtres ; et la pierre.

Le domaine technique de l'invention peut ainsi, de manière générale, être défini comme étant celui de la décontamination de surfaces en vue d'en éliminer les polluants, contaminants qui s'y trouvent et dont la présence sur ces surfaces n'est pas souhaitée.

Plus particulièrement, le domaine technique de l'invention est celui de la décontamination biologique de surfaces contaminées notamment par des espèces biologiques toxiques par exemple de type endospores, toxines, virus.

### ÉTAT DE LA TECHNIQUE ANTÉRIEURE

Depuis une vingtaine d'années, la succession d'actes terroristes chimiques et plus récemment biologiques, par exemple l'attentat au gaz sarin dans le métro de Tokyo en 1995, les attentats suicides sur les Twin Towers de New York en 2001, l'anthrax dans les courriers de l'US Postal aux Etats-Unis en 2001, et les attentats à l'explosif dans les gares de Madrid en 2004, a incité de nombreux pays à bâtir de véritables programmes de recherche pour se munir de moyens efficaces face aux menaces terroristes.

Essentiellement de nature chimique au début du XX^{ieme} siècle, les agents de la menace ont évolué vers des armes de plus forts impacts, plus simples à mettre en oeuvre et surtout non détectables avant l'apparition des premiers symptômes sur l'organisme.

La crainte se porte donc aujourd'hui sur les attaques terroristes de type biologiques particulièrement contagieuses par voie orale. Les espèces biologiques toxiques telles que le Bacillus anthracis (anthrax) ou encore la toxine botulique sont considérées comme les armes dont la probabilité d'utilisation est la plus forte.

Dans le cas de la décontamination biologique, deux objectifs sont recherchés :
- le premier est d'inactiver les contaminants biologiques, bio-toxiques lors du contact prolongé entre l'agent biocide (généralement une espèce chimique fortement oxydante) et l'agent pathogène. Cette phase d'inhibition nécessite une durée de contact pouvant atteindre plusieurs heures selon la formulation utilisée,
- le deuxième est d'essayer, le plus souvent, de transférer les espèces contaminantes vers une phase solide ou liquide permettant l'élimination des espèces inactivées du matériau traité.

D'une façon générale, les techniques d'assainissement des matériaux contaminés par une contamination biologique consistent en la mise en contact d'un liquide contenant un agent biocide avec les surfaces contaminées. L'application de la solution biocide est généralement réalisée par pulvérisation ou par lavage couplé ou non à un effet mécanique tel qu'un brossage.

Ainsi, le document [1] décrit-il une composition de nettoyage pour éliminer les agents antibactériens et autres utilisés dans la décontamination suite à une attaque biologique. Cette composition comprend notamment de l'éthanol, de l'isopropanol, de l'éther n-hexylique de l'éthylèneglycol, un bromure et un chlorure.

Le document [2] décrit un procédé de décontamination à grande échelle dans lequel un peracide solide, stable, ou une source solide, stable, d'un peracide est mis en contact avec une surface contaminée.

Plus récemment, de nouvelles techniques de mouillage par nébulisation ou par projection de mousse ont permis de réduire la quantité de solutions biocides utilisées et donc le volume d'effluents chimiques produits. On pourra à cet égard se référer aux documents [3] et [4].

D'autres procédés utilisent les agents biocides sous forme gazeuse tels que le peroxyde d'hydrogène ou encore l'ozone, comme c'est le cas du procédé décrit dans le document [5].

L'inconvénient majeur associé à ces procédés est toutefois le risque de dissémination d'agents toxiques dans l'environnement, qu'il s'agisse d'agents toxiques biologiques et chimiques dans le cas du procédé par pulvérisation de liquide, ou bien d'agents toxiques chimiques dans le cas du procédé mettant en oeuvre des agents biocides sous forme gazeuse.

En outre, le procédé qui met en oeuvre des agents biocides sous forme gazeuse, est exclusivement efficace lors d'une mise en oeuvre dans des enceintes closes.

Pour répondre à la problématique de récupération de la contamination, une troisième catégorie de procédé a plus récemment été développée.

Dans ces procédés, le transfert de la contamination se fait vers un matériau support solide capable de piéger et/ou de détruire les espèces biologiques toxiques. Le déchet ainsi généré se retrouve alors également sous forme solide. L'obtention d'un déchet solide est particulièrement intéressante pour limiter les risques de dispersion des toxiques dans l'environnement mais aussi pour faciliter la gestion et le traitement du déchet produit.

Différentes technologies mettant en oeuvre un matériau support solide ont d'ores et déjà été développées. Il s'agit : tout d'abord de la technologie dite du « gant poudreur » destinée à la décontamination de liquides toxiques persistants se trouvant sur la peau ou sur des équipements.

Dans ce gant, l'agent décontaminant est une poudre absorbante, généralement de la Terre de foulon. Celle-ci est déversée sur l'endroit contaminé par tapotage, elle absorbe le liquide toxique, puis elle est essuyée à l'aide de la face éponge du gant [6].

La composition du gant peut, dans certains cas, inclure un agent oxydant capable d'inactiver la contamination piégée par la Terre de foulon. Cette technique, particulièrement adaptée au soin des personnes, reste néanmoins limitée au traitement des contaminations liquides de faible envergure.

D'autres produits de décontamination, qui se présentent sous la forme d'un gel, génèrent un déchet solide et permettent ainsi de s'affranchir de l'utilisation de solutions liquides pour assainir des pièces de grandes surfaces et de géométries complexes.

Ces gels sont généralement mis en oeuvre en les pulvérisant sur la surface à décontaminer.

Après un temps de contact du gel avec la surface à décontaminer, équivalent à la durée d'évaporation du solvant, le déchet sec obtenu est éliminé par brossage et/ou aspiration. L'intérêt majeur de ces procédés est leur aptitude au traitement des grandes surfaces et de géométries accidentées.

Ainsi, le document [7] décrit une composition de gel contenant des agents oxydants pour la décontamination chimique ou biologique de zones contaminées. Cette composition est préparée en ajoutant des agents épaississants ou gélifiants sous la forme de colloïdes à une solution d'agent oxydant pour former un gel colloïdal visqueux.

Cette solution peut être une solution aqueuse ou organique.

Les agents épaississants ou gélifiants peuvent être choisis parmi la silice, l'alumine, les aluminosilicates, les mélanges de silice et d'alumine, et les argiles telles que la smectite.

Les agents oxydants sont notamment l'hypochlorite de sodium, le persulfate d'ammonium, ou le peroxyde d'hydrogène.

Il est indiqué que ces gels peuvent être utilisés pour éliminer des agents biologiques tels que des micro-organismes comme les bactéries, champignons, virus, et spores, ou des agents chimiques tels que les gaz neurotoxiques.

Les gels sont ensuite pulvérisés sur les surfaces à traiter puis récupérés par aspiration après séchage.

Il est indiqué qu'un gel oxydant contenant du peroxymonosulfate de potassium et 15% de silice Cab-O-Sil^{®} EH-5 en tant qu'agent gélifiant, détruit les agents chimiques « *Mustard* », « *VX* » et « *GD* » dans le temps nécessaire pour amener le gel à siccité et que le Bacillus globigii (BG), simulant de l'Anthrax est également détruit en partie par ce gel.

Les formulations gélifiées développées par le Lawrence Livermore National Laboratory sous les noms de L-Gel 115, et L-Gel 200 sont analogues aux formulations développées dans le document [7] et sont mises en oeuvre avec le procédé dit « L-Gel ». Ce procédé semble avoir une certaine efficacité vis-à-vis d'une contamination biologique telle qu'une contamination par les spores de Bacillus globigii [8].

Ces gels sont formulés à partir de solutions acides oxydantes auxquelles sont ajoutés des solvants organiques et une charge de silice. Les gels sont ensuite pulvérisés sur les surfaces à traiter puis récupérés par aspiration après séchage. Parmi les points critiques de ce procédé, apparaît en premier lieu la présence d'agents oxydants puissants dont la stabilité chimique est souvent très limitée dans le temps.

Par ailleurs, afin d'éviter les coulures, en particulier lorsque le gel est appliqué sur les murs ou les plafonds, celui ci est appliqué sous forme de films très minces d'une épaisseur ne dépassant pas, dans le document [7], 125 µm. Il en résulte un déchet sec pulvérulent pouvant entraîner, si l'efficacité du traitement n'est pas totale, une dissémination des espèces bio-toxiques et chimiques, telles que les composés oxydants, dans l'atmosphère.

Les performances du procédé, déterminées vis-à-vis d'une contamination par l'Anthrax sous forme d'aérosol (10⁷ et 10⁸ spores par échantillon de 0,16 m²), montrent qu'il n'autorise pas une réduction de la contamination supérieure à 4 décades [8].

Par ailleurs, dans le cadre de la décontamination nucléaire, des formulations gélifiées qui permettent de s'affranchir des problèmes liés au caractère pulvérulent du déchet sec et d'accroître l'efficacité du procédé gel ont fait l'objet des documents [9] et [10]. Les formulations de gel qui y sont décrites permettent de libérer, au moyen d'une faible érosion du matériau support, la contamination sous forme particulaire. Le contaminant se retrouve alors piégé dans le résidu de gel sec [9], [10].

Ces gels sont spécifiquement destinés à la décontamination radioactive et ne sont, en aucune manière, adaptés ou susceptibles d'être adaptés à la décontamination biologique de surfaces.

En outre, les gels décrits plus haut ne permettent pas la décontamination en profondeur de matériau poreux.

Il existe donc, au regard de ce qui précède, un besoin pour un gel de décontamination biologique qui produise des déchets secs faciles à éliminer sans dissémination des contaminants biologiques, qui permette de traiter avec la même efficacité une grande variété de surfaces quelles que soient leur forme, leur géométrie, leur taille et leur nature.

Il existe, en particulier, un besoin pour un gel de décontamination biologique qui permette la décontamination efficace de surfaces poreuses notamment minérales et qui assure la décontamination en profondeur de telles surfaces sur une profondeur pouvant atteindre par exemple plusieurs millimètres.

Il existe encore un besoin pour un gel de décontamination qui ne produise aucune altération, chimique, mécanique ou physique des surfaces traitées.

Le but de la présente invention est de fournir un gel de décontamination biologique qui réponde entre autres à ce besoin.

Le but de la présente invention est encore de fournir un gel de décontamination qui ne présente pas les inconvénients défauts, limitations et désavantages des gels de décontamination de l'art antérieur et qui résolve les problèmes des gels de décontamination de l'art antérieur.

### EXPOSÉ DE L'INVENTION

Ce but, et d'autres encore, sont atteints, conformément à l'invention, par un gel de décontamination biologique, constitué par une solution colloïdale comprenant, de préférence constituée par :
- 5 à 30% en masse, de préférence 5 à 25% en masse, de préférence encore 8 à 20% en masse par rapport à la masse du gel, d'au moins un agent viscosant inorganique ;
- 0,5 à 10 mol/L de gel, de préférence 1 à 10 mol/L de gel, d'au moins un agent actif de décontamination biologique ;
- 0,05 à 5% en masse, de préférence 0,05 à 2% en masse par rapport à la masse du gel, d'au moins un polymère super-absorbant ;
- 0,1 à 2% en masse par rapport à la masse du gel, d'au moins un agent tensio-actif ;
- et le reste de solvant.

Les gels selon l'invention n'ont jamais été décrits dans l'art antérieur.

En particulier, l'incorporation d'un polymère super-absorbant dans un gel de décontamination et a fortiori la combinaison dans un tel gel d'un tel polymère super-absorbant avec un agent de décontamination biologique n'ont jamais été décrites dans l'art antérieur, tel que représenté notamment par les documents cités plus haut.

Les gels selon l'invention répondent à l'ensemble des besoins mentionnés plus haut, ils ne présentent pas les inconvénients, défauts, limitations et désavantages des gels de l'art antérieur tels que ceux décrits dans les documents mentionnés plus haut.

Les gels selon l'invention résolvent les problèmes présentés par les gels de décontamination biologique de l'art antérieur sans en présenter les inconvénients mais tout en conservant toutes les propriétés avantageuses connues de ces gels.

Le gel selon l'invention est une solution colloïdale, ce qui signifie que le gel selon l'invention contient des particules solides inorganiques, minérales, d'agent viscosant dont les particules élémentaires, primaires, ont une taille généralement de 2 à 200 nm.

Du fait de la mise en oeuvre d'un agent viscosant généralement exclusivement inorganique, sans agent viscosant organique, la teneur en matières organiques du gel selon l'invention est généralement inférieure à 4% en masse, de préférence inférieure à 2% en masse, ce qui constitue encore un autre avantage des gels selon l'invention.

Ces particules solides, minérales, inorganiques jouent le rôle de viscosant pour permettre à la solution, par exemple la solution aqueuse, de se gélifier et ainsi d'adhérer aux surfaces à traiter, décontaminer, quelle que soit leur géométrie, leur forme, leur taille, et où que se trouvent les contaminants à éliminer.

Avantageusement, l'agent viscosant inorganique peut être choisi parmi les alumines, les silices, les aluminosilicates, les argiles telles que la smectite, et leurs mélanges.

En particulier, le viscosant inorganique peut être choisi parmi les alumines (Al₂O₃) et les silices (SiO₂).

Le viscosant inorganique peut ne comprendre qu'une seule silice ou alumine ou un mélange de celles-ci, à savoir un mélange de deux silices différentes ou plus (mélange SiO₂/SiO₂), un mélange de deux alumines, différentes ou plus (mélange Al₂O₃/Al₂O₃), ou encore un mélange d'une ou plusieurs silices avec une ou plusieurs alumines (mélange SiO₂/Al₂O₃).

Avantageusement, l'agent viscosant inorganique peut être choisi parmi les silices pyrogénées, les silices précipitées, les silices hydrophiles, les silices hydrophobes, les silices acides, les silices basiques comme la silice Tixosil 73 (marque de commerce) commercialisée par la société Rhodia, et leurs mélanges.

Parmi les silices acides, on peut notamment citer les silices pyrogénées ou fumées de silice "Cab-O-Sil" M5, H5 ou EH5 (marques de commerce) commercialisées par la société CABOT, et les silices pyrogénées commercialisées par la société DEGUSSA sous l'appellation AEROSIL (marques de commerce).

Parmi ces silices pyrogénées, on préférera encore la silice AEROSIL 380 (marque de commerce) d'une surface spécifique de 380 m²/g qui offre les propriétés viscosantes maximales pour une charge minérale minimale.

La silice utilisée peut aussi être une silice dite précipitée obtenue par exemple par voie humide par mélange d'une solution de silicate de soude et d'un acide. Les silices précipitées préférées sont commercialisées par DEGUSSA sous le nom de SIPERNAT 22 LS et FK 310 (marques de commerce) ou encore par la société RHODIA sous le nom de TIXOSIL 331 (marque de commerce), cette dernière est une silice précipitée dont la surface spécifique moyenne est comprise entre 170 et 200 m²/g.

Avantageusement, l'agent viscosant inorganique est constitué par un mélange d'une silice précipitée et d'une silice pyrogénée.

L'alumine peut être choisie parmi les alumines calcinées, les alumines calcinées broyées, et leurs mélanges.

A titre d'exemple, on peut citer le produit vendu par DEGUSSA sous la désignation commerciale « Aeroxide Alumine C » qui est de l'alumine fine pyrogénée.

De manière avantageuse, selon l'invention, l'agent viscosant est constitué par une ou plusieurs alumine(s) représentant généralement de 5% à 30% en masse par rapport à la masse du gel.

Dans ce cas, l'alumine est de préférence à une concentration de 8 à 17% en masse par rapport à la masse totale du gel pour assurer un séchage du gel à température comprise entre 20°C et 50°C et à une humidité relative comprise entre 20 et 60% en moyenne en 30 minutes à 5 heures.

La nature de l'agent viscosant minéral, notamment lorsqu'il est constitué d'une ou plusieurs alumine(s), influence de manière inattendue le séchage du gel selon l'invention et la granulométrie du résidu obtenu.

En effet, le gel sec se présente sous la forme de particules de taille contrôlée, plus précisément de paillettes solides millimétriques, dont la taille va généralement de 1 à 10 mm de préférence de 2 à 5 mm grâce notamment aux compositions précitées de la présente invention, en particulier lorsque l'agent viscosant est constitué par une ou plusieurs alumine (s) .

Précisons que la taille des particules correspond généralement à leur plus grande dimension.

Le gel selon l'invention contient un agent actif de décontamination biologique.

Par agent de décontamination biologique que l'on peut aussi qualifier d'agent biocide, on entend tout agent, qui lorsqu'il est mis en contact avec une espèce biologique et notamment une espèce biologique toxique est susceptible, d'inactiver ou de détruire celle-ci.

Par espèce biologique, on entend tout type de micro-organisme tel que les bactéries, les champignons, les levures, les virus, les toxines, les spores notamment les spores de Bacillus Anthracis, et les protozoaires.

Les espèces biologiques qui sont éliminées, détruites, inactivées, par le gel selon l'invention sont essentiellement des espèces bio-toxiques telles que les spores pathogènes comme par exemple les spores de Bacillus Anthracis, les toxines comme par exemple la toxine botulique, et les virus.

L"agent actif de décontamination biologique peut être choisi parmi les bases telles que l'hydroxyde de sodium, l'hydroxyde de potassium, et leurs mélanges ; les acides tels que l'acide nitrique, l'acide phosphorique, l'acide chlorhydrique, l'acide sulfurique, et leurs mélanges ; les agents oxydants tels que les peroxydes, permanganates, persulfates, l'ozone, les hypochlorites, et leurs mélanges ; les sels d'ammonium quaternaires tels que les sels d'hexacétylpyridinium comme le chlorure d'hexacéthylpyridinium ; et leurs mélanges (voir notamment les Exemples 1 et 2).

Certains agents actifs de décontamination peuvent être classés parmi plusieurs des catégories définies plus haut.

Ainsi, l'acide nitrique est-il un acide mais aussi un agent oxydant.

L'agent actif de décontamination, tel qu'un agent biocide, est généralement utilisé à une concentration comprise entre 0,5 et 10 mol/L de gel, et de préférence de 1 à 10 mol/L de gel afin de garantir un pouvoir d'inhibition des espèces biologiques, notamment biotoxiques, compatible avec le temps de séchage du gel et pour assurer par exemple un séchage du gel à une température comprise entre 20°C et 50°C et à une humidité relative comprise entre 20 et 60 % en moyenne en 30 minutes à 5 heures.

L'agent actif de décontamination peut être un acide ou un mélange d'acides. Ces acides sont généralement choisis parmi les acides minéraux tels que l'acide chlorhydrique, l'acide nitrique, l'acide sulfurique et l'acide phosphorique.

Un agent décontaminant biologique particulièrement préféré est l'acide nitrique.

En effet, il s'est avéré de manière totalement surprenante que l'acide nitrique détruisait, inactivait, les espèces biologiques notamment biotoxiques.

En particulier, il a été mis en évidence de manière étonnante que l'acide nitrique assurait la destruction, l'inactivation, des spores telles que les spores de Bacillus thuringiensis qui sont des espèces particulièrement résistantes.

L'acide ou les acides est(sont) de préférence présent (s) à une concentration de 0,5 à 10 mol/L, de préférence encore de 1 à 10 mol/L, pour assurer un séchage du gel généralement à une température comprise entre 20°C et 50°C et à une humidité relative comprise entre 20 et 60% en moyenne en 30 minutes à 5 heures.

Pour ce type de gel acide, l'agent viscosant inorganique est de préférence la silice ou un mélange de silices.

Ou bien, l'agent actif de décontamination biologique peut être une base de préférence une base minérale, choisie de préférence parmi la soude, la potasse, et leurs mélanges.

Dans le cas d'une telle formulation de gel basique, le gel selon l'invention a, outre l'action de décontamination, une action de dégraissage.

De manière à atteindre une efficacité totale, y compris dans les conditions climatiques les plus défavorables vis-à-vis du temps de séchage du gel, le gel selon invention peut présenter une large gamme de concentration en agent(s) de décontamination basique (s) .

En effet, l'augmentation de la concentration en agent de décontamination basique comme NaOH ou KOH, jouant généralement le rôle d'agent biocide, permet d'accroître considérablement les vitesses d'inhibition des espèces biologiques, comme cela a été démontré pour des spores de Bacillus thuringiensis (Exemple 2).

La base est avantageusement présente à une concentration inférieure à 10 mol/L, de préférence entre 0,5 et 7 mol/L, de préférence encore entre 1 et 5 mol/L pour assurer un séchage du gel à température comprise entre 20°C et 50°C et humidité relative comprise entre 20 et 60% en moyenne en 30 minutes à 5 heures.

Pour ce type de gel alcalin, basique, l'agent viscosant inorganique est de préférence une alumine ou un mélange d'alumines.

De manière à aboutir à l'efficacité maximale sur une large gamme de matériaux tout en garantissant l'innocuité du traitement, l'agent de décontamination biologique est de préférence l'hydroxyde de sodium ou l'hydroxyde de potassium.

Dans le cas du traitement d'une matrice cimentaire, le pH basique du gel, qui est induit par l'utilisation de la soude ou de la potasse, permet d'éviter les réactions acido-basiques, entre le matériau à décontaminer et le gel, qui nuisent à l'intégrité du gel sur la surface et donc à l'efficacité du procédé.

Le caractère hygroscopique de l'hydroxyde de sodium ou de l'hydroxyde de potassium constitue également un atout considérable pour ralentir le phénomène de séchage du gel. Le temps de contact entre le gel selon l'invention, contenant par exemple une solution biocide, et la contamination biologique, s'en retrouve alors considérablement augmenté.

En effet, la compétition entre le processus d'évaporation de la phase aqueuse et celui de reprise d'eau des cristaux d'hydroxyde de sodium ou d'hydroxyde de potassium modifie favorablement la cinétique de séchage du gel (Exemple 3).

Au regard de la cinétique d'inhibition des spores (Exemple 2) et des durées de séchage des gels en fonction de la température (Exemple 4), l'agent biocide sera de préférence l'hydroxyde de sodium à une concentration comprise entre 1 et 5 mol/L.

Le gel selon l'invention contient en outre en tant que composant fondamental un polymère super-absorbant.

Par « polymère super-absorbant » également dénommé « SAP », on entend généralement un polymère capable, à l'état sec, d'absorber spontanément au moins 10 fois, de préférence au moins 20 fois son poids de liquide aqueux, en particulier d'eau et notamment d'eau distillée.

Certains « SAP » peuvent absorber jusqu'à et même plus de 1000 fois leur poids de liquide.

De tels polymères super-absorbants sont notamment décrits dans l'ouvrage « Absorbent Polymer Technology, Studies in Polymer Science 8 » de L. BRANNON-PAPPAS et R. HARLAND, édition Elsevier, 1990, auquel on pourra se référer.

Par absorption spontanée, on entend un temps d'absorption allant jusqu'à environ une heure.

Le polymère super-absorbant peut avoir une capacité d'absorption d'eau allant de 10 à 2000 fois son propre poids, de préférence de 20 à 2000 fois son propre poids (soit 20 g à 2000 g d'eau absorbée par gramme de polymère absorbant), de préférence encore de 30 à 1500 fois, et en particulier de 50 à 1000 fois.

Ces caractéristiques d'absorption d'eau s'entendent dans les conditions normales de température (25°C) et de pression (760 mm Hg soit 100000 Pa) et pour de l'eau distillée.

Le SAP du gel de décontamination biologique selon l'invention peut être choisi parmi les poly(méth)acrylates de sodium, les amidons greffés par un polymère (méth)acrylique, les amidons hydrolysés greffés par un polymère (méth)acrylique ; les polymères à base d'amidon, de gomme, et de dérivé cellulosique ; et leurs mélanges.

Plus précisément, le SAP utilisable dans le gel selon l'invention peut être par exemple choisi parmi :
- les polymères résultants de la polymérisation avec réticulation partielle de monomères à insaturation éthylénique hydrosolubles, tels que les polymères acryliques, méthacryliques (issus notamment de la polymérisation de l'acide acrylique et/ou méthacrylique et/ou de monomères acrylate et/ou méthacrylate) ou vinyliques, en particulier les poly(méth)acrylates réticulés et neutralisés, notamment sous forme de gel ; et les sels notamment les sels alcalins tels que les sels de sodium ou de potassium de ces polymères ;
- les amidons greffés par des polyacrylates ;
- les copolymères acrylamide/acide acrylique, notamment sous forme de sels de sodium ou de potassium ;
- les amidons greffés acrylamide/acide acrylique, notamment sous forme de sels de sodium ou de potassium ;
- les sels de sodium ou de potassium de carboxyméthylcellulose ;
- les sels notamment les sels alcalins, d'acides polyaspartiques réticulés ;
- les sels notamment les sels alcalins, d'acides polyglutamiques réticulés.

En particulier, on peut utiliser comme « SAP » un composé choisi parmi :
- les polyacrylates de sodium ou de potassium réticulés vendus sous les dénominations SALSORB CL 10, SALSORB CL 20, FSA type 101, FSA type 102 (Allied Colloids) ; ARASORB S-310 (Arakawa Chemical) ; ASAP 2000, Aridall 1460 (Chemdal) ; KI-GEL 201-K (Siber Hegner) ; AQUALIC CA W3, AQUALIC CA W7, AQUALIC CA W10; (Nippon Shokuba) ; AQUA KEEP D 50, AQUA KEEP D 60, AQUA KEEP D 65, AQUA KEEP S 30, AQUA KEEP S 35, AQUA KEEP S 45, AQUA KEEP Al M1, AQUA KEEP A1 M3, AQUA KEEP HP 200, , NORSOCRYL S 35, NORSOCRYL FX 007 (Arkema) ; AQUA KEEP 10SH-NF, AQUA KEEP J-550 (Kobo); LUQUASORB CF, LUQUASORB MA 1110, LUQUASORB MR 1600, HYSORB C3746-5 (BASF) ; COVAGEL (Sensient technologies), SANWET IM-5000D (Hoechst Celanese) ;
- les polyacrylates greffés d'amidon vendus sous les dénominations SANWET IM-100, SANWET IM-3900, SANWET In-5000S (Hoechst) ;
- les copolymères acrylamide/acide acrylique greffés d'amidon sous forme de sel de sodium ou de potassium vendus sous les dénominations WATERLOCK A- 100, WATERLOCK A-200, WATERLOCK C-200, WATERLOCK D-200, WATERLOCK B-204 (Grain Processing Corporation) ;
- les copolymères acrylamide/acide acrylique sous forme de sel de sodium vendu sous la dénomination WATERLOCK G-400 (Grain Processing Corporation) ;
- la carboxyméthylcellulose vendue sous les dénominations AQUASORB A250 (Aqualon) ;
- le polyglutamate de sodium réticulé vendu sous la dénomination GELPROTEIN (Idemitsu Technofine).

Les polymères super-absorbants, en particulier les polymères super-absorbants (polyélectrolytes) qui contiennent des ions alcalins tels que des ions sodium ou potassium, par exemple de type poly(méth)acrylate de sodium ou de potassium, confèrent de nombreuses propriétés aux gels de décontamination.

Ils influencent tout d'abord la rhéologie du produit, notamment son seuil d'écoulement. En terme de mise en oeuvre du procédé, l'intérêt est de garantir une tenue parfaite du gel sur les matériaux traités, notamment sur les surfaces verticales et les plafonds lorsque l'épaisseur de gel pulvérisé est supérieure à 1 mm.

Dans le cadre d'un procédé de décontamination biologique par gel, le polymère super-absorbant est particulièrement intéressant car il absorbe par liaison hydrogène une partie de la solution, par exemple de la solution biocide contenue dans le gel. Le nombre de liaisons hydrogènes formées entre la solution, par exemple la solution biocide, du gel et le polymère super-absorbant tel que le polyacrylate de sodium étant fonction de la charge saline, des phénomènes d'absorption/désorption apparaissent lorsque la charge saline du gel de décontamination est modifiée.

Ce mécanisme est alors particulièrement intéressant lorsqu'il s'agit de décontaminer des matériaux minéraux et poreux comme les matrices cimentaires par exemple.

En effet, au contact du matériau, la charge saline du gel augmente du fait de la présence de particules minérales très souvent à base de calcium. Au sein du polymère super absorbant tel que le polyacrylate de sodium, la substitution du contre-ion Na⁺ par Ca²⁺ issu du calcium génère instantanément un phénomène de re-larguage de solution, par exemple de solution biocide, en raison de l'encombrement stérique plus important de l'ion calcium.

La quantité de solution biocide libérée par le polymère super-absorbant tel que le polyacrylate de sodium peut alors diffuser instantanément dans la porosité du matériau et pénétrer en profondeur.

Le phénomène de diffusion de l'agent de décontamination, par exemple de l'agent biocide vers le coeur du matériau est beaucoup plus limité dans le cas d'un gel ne contenant pas de super-absorbant (voir l'Exemple 6).

L'ajout de polymère super-absorbant au gel selon l'invention permet donc d'accroître significativement l'efficacité du gel et du procédé selon l'invention en présence de matériaux poreux contaminés en profondeur sur une épaisseur de un à plusieurs millimètres, par exemple jusqu'à 2, 5, 10, 20 voire 100 mm (Exemple 6).

Le polymère super-absorbant peut être choisi de préférence parmi les gammes Aquakeep^{®} ou Norsocryl^{®} commercialisées par la société ARKEMA.

Le gel peut aussi contenir un agent tensio-actif ou un mélange d'agents tensio-actifs, de préférence choisis parmi la famille des agents tensio-actifs non ioniques tels que les copolymères blocs, séquencés comme les copolymères séquencés d'oxyde d'éthylène et d'oxyde de propylène, et les acides gras éthoxylés, et leurs mélanges.

Pour ce type de gel, les agents tensio-actifs sont de préférence des copolymères blocs commercialisés par la société BASF sous la dénomination "PLURONIC^{®}".

Les Pluronics^{®} sont des copolymères séquencés d'oxyde d'éthylène et d'oxyde de propylène.

Ces agents tensio-actifs influencent les propriétés rhéologiques du gel, notamment le caractère thixotropique du produit et le temps de reprise, afin de le rendre pulvérisable aussi bien sur les planchers, les murs ou les plafonds en évitant l'apparition de coulure.

Les tensio-actifs permettent, par ailleurs, de maîtriser l'adhésion du déchet sec [Exemple 7] et de contrôler la taille des paillettes de résidu sec pour garantir la non-pulvérulence du déchet [Exemple 8].

Le solvant selon l'invention est généralement choisi parmi l'eau, les solvants organiques, et leurs mélanges.

Un solvant préféré est l'eau, et dans ce cas, le solvant est donc constitué par de l'eau, comprend 100% d'eau.

L'invention concerne, en outre, un procédé de décontamination biologique d'une surface d'un substrat solide contaminée par au moins une espèce biologique se trouvant sur ladite surface et éventuellement sous ladite surface dans la profondeur du substrat, dans lequel on réalise au moins un cycle comprenant les étapes successives suivantes :
a) on applique le gel selon l'invention tel que décrit plus haut sur ladite surface;
b) on maintient le gel sur la surface au moins pendant une durée suffisante pour que le gel détruise et/ou inactive et/ou absorbe l'espèce biologique, et pour que le gel sèche et forme un résidu sec et solide contenant ladite espèce biologique ;
c) on élimine le résidu sec et solide contenant ladite espèce biologique.

Il est à noter que, dans le cas d'une surface non poreuse, la contamination biologique « inactivée » est récupérée par les paillettes de gel sec.

Par contre dans le cas d'une contamination profonde, comme c'est le cas dans les matériaux poreux tels que les matrices cimentaires, le gel sec ne contiendra que le résidu de contamination surfacique.

La contamination interne, profonde, « inactivée » *in situ* suite à l'action du super-absorbant, du gel restera au coeur du matériau, substrat.

Avantageusement, le substrat solide est un substrat poreux, de préférence un substrat minéral poreux.

Toutefois, l'efficacité du gel et du procédé selon l'invention est tout aussi bonne en présence d'une surface non poreuse et/ou non minérale.

Avantageusement, le substrat est en au moins un matériau choisi parmi les métaux comme l'acier inoxydable ; les polymères tels que les matières plastiques ou caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments ; les mortiers et bétons ; les plâtres ; les briques ; la pierre naturelle ou artificielle ; les céramiques.

Avantageusement, l'espèce biologique est choisie parmi les espèces biologiques toxiques déjà énumérées plus haut.

Avantageusement, le gel est appliqué sur la surface à décontaminer à raison de 100 g à 2000 g de gel par m² de surface, de préférence de 500 à 1500 g de gel par m² de surface, de préférence encore de 600 à 1000 g par m² de surface, ce qui correspond généralement à une épaisseur de gel déposé sur la surface comprise entre 0,5 mm et 2 mm.

Avantageusement, le gel est appliqué sur la surface solide par pulvérisation, au pinceau ou avec une taloche.

Avantageusement (lors de l'étape b)), le séchage est réalisé à une température de 1°C à 50°C, de préférence de 15°C à 25°C, et sous une humidité relative de 20% à 80%, de préférence de 20% à 70%.

Avantageusement, le gel est maintenu sur la surface pendant une durée de 2 à 72 heures, de préférence de 2 à 48 heures, de préférence encore de 5 à 24 heures.

Avantageusement, le résidu sec et solide se présente sous la forme de particules, par exemple de paillettes, d'une taille de 1 à 10 mm, de préférence de 2 à 5 mm.

Avantageusement, le résidu sec et solide est éliminé de la surface solide par brossage et/ou aspiration.

Avantageusement, le cycle décrit plus haut peut être répété par exemple de 1 à 10 fois en utilisant le même gel lors de tous les cycles ou en utilisant des gels différents lors d'un ou de plusieurs cycle(s).

Avantageusement, lors de l'étape b), le gel, avant séchage total, est remouillé avec une solution d'un agent de décontamination biologique, de préférence avec la solution de l'agent actif biologique du gel appliqué lors de l'étape a) dans le solvant de ce gel.

Lors de l'étape b), le gel peut avant séchage total être remouillé avec la solution biocide contenue dans le gel de décontamination biologique déjà décrit plus haut, ce qui évite alors généralement de répéter l'application du gel sur la surface et occasionne une économie de réactif et une quantité de déchet limitée. Cette opération de remouillage peut être répétée.

En résumé, le procédé et le gel selon l'invention présentent entre autres les propriétés avantageuses suivantes :
- l'application du gel par pulvérisation,
- l'adhérence aux parois,
- l'obtention de l'efficacité maximale de décontamination à l'issue de la phase de séchage du gel, y compris en situation de contamination pénétrante notamment dans le cas de surfaces poreuses.

En général, on fait en sorte que le temps de séchage soit supérieur ou égal à la durée nécessaire pour l'inactivation. Dans le cas d'une inactivation profonde, on fait généralement appel à un remouillage.
- le traitement d'une gamme très large de matériaux,
- l'absence d'altération mécanique ou physique des matériaux à l'issue du traitement,
- la mise en oeuvre du procédé dans des conditions climatiques variables,
- la réduction du volume de déchet,
- la facilité de récupération du déchet sec.

D'autres caractéristiques et avantages de l'invention apparaîtront mieux à la lecture de la description détaillée qui suit, cette description étant faite à titre illustratif et non limitatif, en liaison avec les dessins joints.

### BRÈVE DESCRIPTION DES DESSINS.

- La Figure 1 (A, B) présente des vues en coupe schématique illustrant les étapes principales du procédé selon l'invention pour la décontamination d'un matériau solide.
- La Figure 2 (A, B, C) présente des vues en coupe schématique montrant le mode d'action d'un gel exempt de polymère super-absorbant sur un matériau cimentaire contaminé en profondeur par une contamination sous forme liquide.

- La Figure 3 (A, B, C) présente des vues en coupe schématique montrant le mode d'action d'un gel contenant un polymère super-absorbant sur un matériau cimentaire contaminé en profondeur par une contamination sous forme liquide.

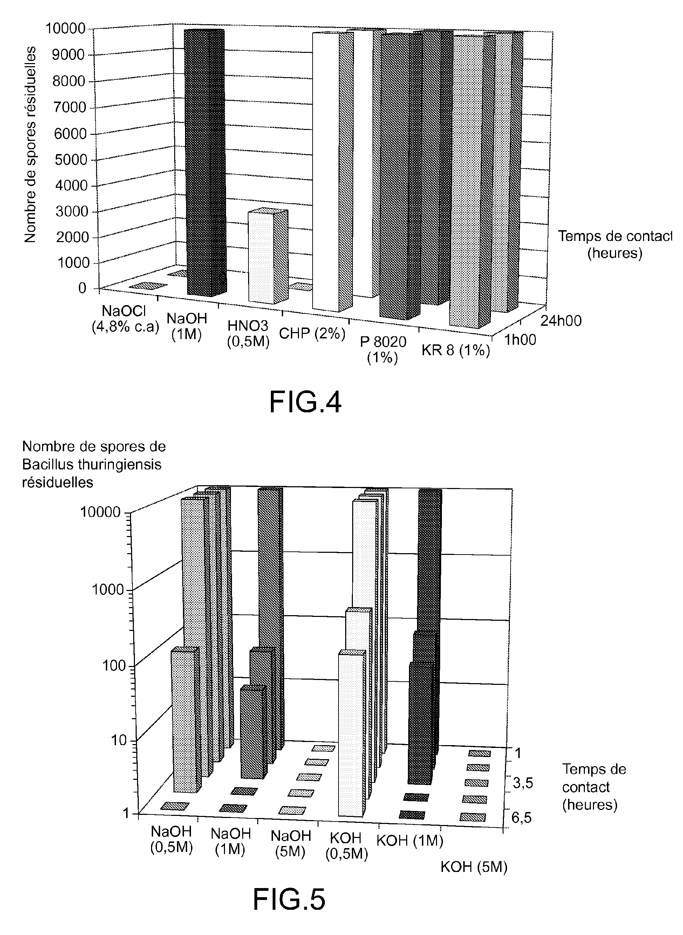

- La Figure 4 est un graphique qui représente la cinétique d'inhibition des spores de Bacillus thuringiensis, dans différentes solutions biocides liquides contenant différents agents actifs de décontamination à diverses concentrations, à savoir : NaOCl à 4, 8%, NaOH à 1M, HNO₃ à 0, 5M, et CHP (chlorure d'hexadecyl pyridinium) à 2% ; des solutions comparatives contenant le tensio-actif Pluronic^{®} P 8020 à 1%, ou le tensio-actif KR8 (alcool gras éthoxylé) à 1% sont également testées. Le nombre de spores résiduelles est donné pour chacune des solutions biocides à des temps de contact de 1 heure et 24 heures.
- La Figure 5 est un graphique qui représente la cinétique d'inhibition des spores de Bacillus thuringiensis, dans différentes solutions biocides liquides contenant différentes bases à diverses concentrations, à savoir : NaOH à 0,5 M, NaOH à 1M, NaOH à 5M, KOH à 0,5M, KOH à 1M, et KOH à 5M. Le nombre de spores résiduelles est donné pour chacune des solutions biocides à des temps de contact de 1 heure, 2 heures, 3 heures, 4 heures et 5 heures.
- La Figure 6 est un graphique qui représente l'influence de la concentration en hydroxyde de sodium dans le gel sur la durée de séchage.
   En ordonnée est portée la perte de masse du gel en % et en abscisse est porté le temps de séchage du gel en jours.
   Les courbes A, B, C, et D représentent respectivement le séchage de gel sans NaOH (uniquement de l'eau), et avec des concentrations en NaOH de 1M, 5M et 10M.
- La Figure 7 est un graphique qui représente l'influence de la température sur la cinétique de séchage d'un gel à base de NAOH 1M ; et la cinétique de séchage d'un gel à base de KOH 1M.
   En ordonnée est portée la perte de masse du gel en % et en abscisse est porté le temps de séchage du gel en minutes.
   La courbe A représente le séchage d'un gel à base de NaOH 1M à 22°C et sous une humidité relative de 40%, la courbe B représente le séchage d'un gel à base de KOH 1M à 22°C et sous une humidité relative de 40%, la courbe C représente le séchage d'un gel à base de KOH 1M à 50°C et sous une humidité relative de 40%.

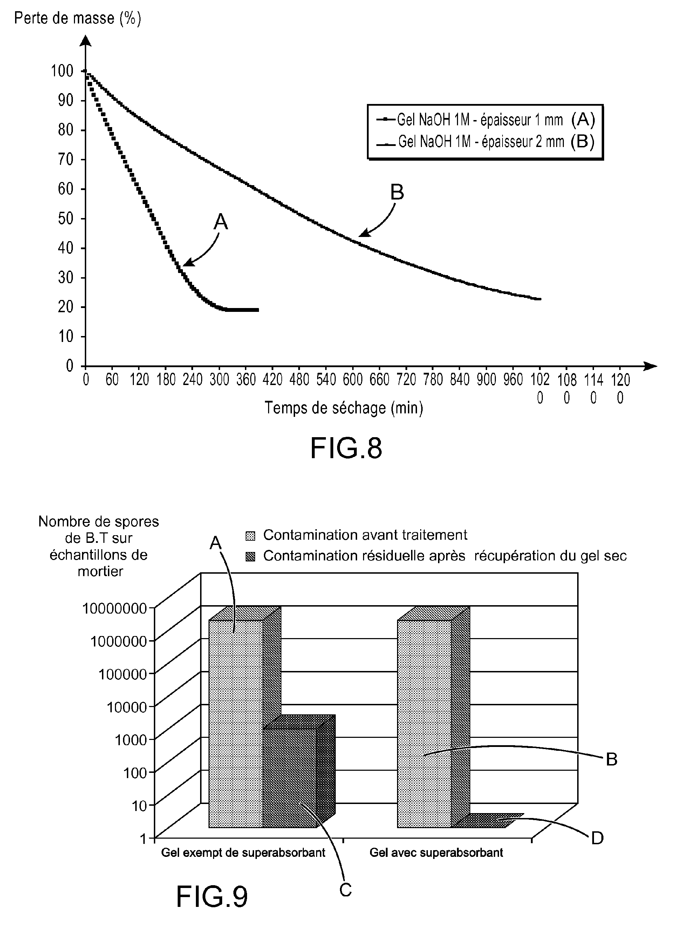

- La Figure 8 est un graphique qui représente l'influence de l'épaisseur de gel déposé sur la cinétique de séchage d'un gel à base de NaOH 1M.
   En ordonnée est portée la perte de masse du gel en % et en abscisse est porté le temps de séchage du gel en minutes.
   La courbe A représente le séchage d'un gel déposé sur une épaisseur de 1 mm, et la courbe B représente le séchage d'un gel déposé sur une épaisseur de 2 mm.
- La Figure 9 est un graphique qui représente l'influence du polymère super-absorbant sur l'efficacité de la décontamination biologique d'un mortier exprimée par le nombre de spores de Bacillus thuringiensis sur un échantillon de mortier.
   Pour chaque gel, les barres de gauche (en gris clair A et B) représentent la contamination des échantillons de mortier avant traitement, et les barres de droite (en gris foncé C et D) représentent la contamination résiduelle des échantillons de mortier après la récupération du gel sec.
   Le graphique présente deux traitements par gel distincts, le premier (partie gauche du graphique, barres A et C accolées à gauche du graphique) en présence d'un gel biocide exempt de polymère super-absorbant, le deuxième (partie droite du graphique, barres B et D accolées à droite du graphique) en présence du même gel biocide auquel a été rajouté le polymère super-absorbant.

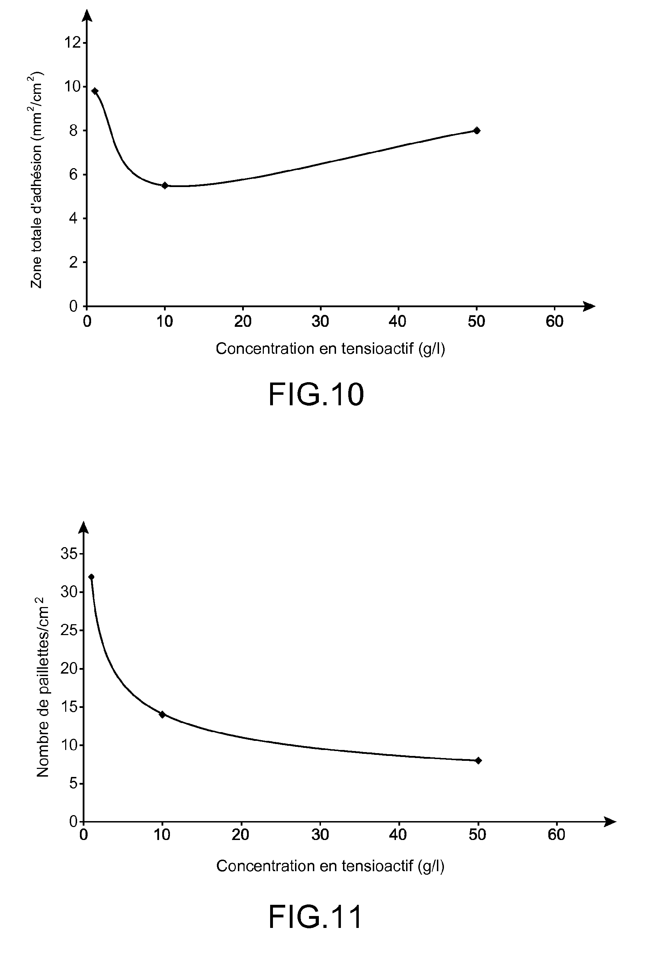

- La Figure 10 est un graphique qui représente l'influence de la concentration en tensio-actif (Pluronic^{®}) sur le pouvoir d'adhésion des paillettes de gel sec.
   En ordonnée est portée la zone totale d'adhésion (mm²/cm²), et en abscisse est portée la concentration en tensio-actif (g/L).
- La Figure 11 est un graphique qui représente l'influence de la concentration en tensio-actif (Pluronic^{®}) sur le nombre de paillettes de gel sec formées.
   En ordonnée est portée le nombre de paillettes/cm², et en abscisse est portée la concentration en tensio-actif (g/L).

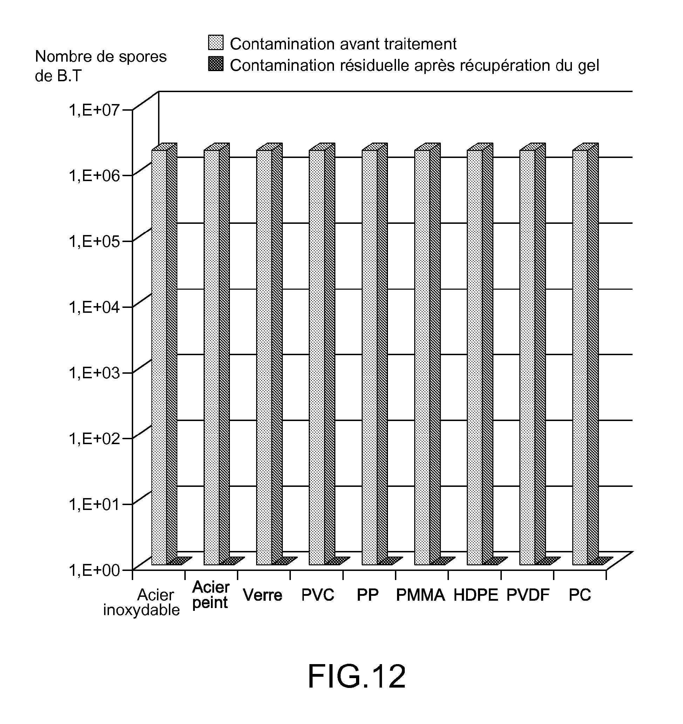

- La Figure 12 est un graphique qui illustre l'efficacité du gel selon l'invention en fonction de la nature du matériau traité.

En ordonnée est porté le nombre de spores de Bacillus thuringiensis.

Pour chaque matériau, les barres de gauche (gris clair) représentent la contamination avant traitement par le gel selon l'invention et les barres de droite (noir) représentent la contamination résiduelle après récupération du gel.

### EXPOSÉ DÉTAILLÉ DE MODES DE RÉALISATION PARTICULIERS

Le gel selon l'invention peut être facilement préparé à la température ambiante.

Par exemple, le gel selon l'invention peut être préparé en ajoutant de préférence progressivement, le ou les agent(s) viscosant(s) inorganique(s) par exemple la ou les alumine (s) et/ou la ou les silice (s), à une solution contenant le ou les agents(s) actif(s) de décontamination biologique(s), le ou les tensioactif(s) et le ou les polymère(s) super-absorbant(s).

Cette addition peut être réalisée en versant simplement le ou les agent (s) viscosant (s) dans ladite solution. Lors de l'addition du ou des agent(s) viscosant (s) inorganique(s), la solution contenant le ou les agent(s) actif(s) de décontamination biologique(s), le ou les tensioactif(s) et le ou les polymère(s) super-absorbant(s) est généralement maintenue sous agitation mécanique. Cette agitation peut être, par exemple, réalisée au moyen d'un agitateur mécanique équipé d'une hélice à trois pales.

La vitesse d'agitation est généralement comprise entre 600 et 800 tours/minute.

Après la fin de l'ajout du ou des viscosant(s) minéral(aux), l'agitation est encore poursuivie, par exemple pendant 2 à 5 minutes, de manière à obtenir un gel parfaitement homogène.

Il est bien évident que d'autres protocoles de préparation des gels selon l'invention peuvent être mis en oeuvre avec une addition des composants du gel dans un ordre différent de celui mentionné plus haut.

Généralement, le gel selon l'invention doit présenter une viscosité inférieure à 200 mPa.s sous un cisaillement de 1000s⁻¹ de manière à permettre la pulvérisation sur la surface à décontaminer, à distance (par exemple à une distance de 1 à 5 m) ou à proximité (par exemple à une distance inférieure à 1 m, de préférence de 50 à 80 cm). Le temps de reprise de la viscosité doit généralement être inférieur à une seconde et la viscosité sous faible cisaillement supérieur à 10 Pa.s pour ne pas couler sur la paroi.

Il est à noter que l'agent tensio-actif du gel selon l'invention influence favorablement et notablement les propriétés rhéologiques du gel selon l'invention. Ce tensio-actif permet notamment que le gel selon l'invention puisse être mis en oeuvre par pulvérisation et évite les risques d'épandage ou de coulure lors du traitement des surfaces verticales et des plafonds.

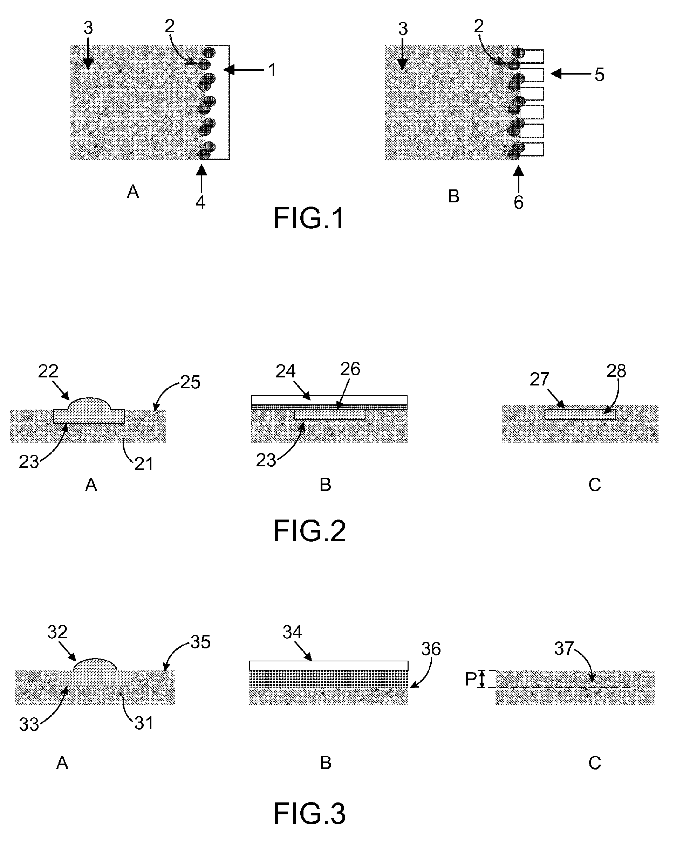

Le gel selon l'invention ainsi préparé est ensuite appliqué (1) (Figure 1) sur la surface solide (2) à décontaminer d'un substrat en un matériau solide (3), en d'autres termes sur la surface (2) ayant été exposée à une contamination biologique (4) ; cette contamination biologique (4) peut être constituée par une ou plusieurs des espèces biologiques déjà définies plus haut.

Comme on l'a déjà indiqué plus haut, l'agent actif de décontamination biologique est choisi en fonction de l'espèce biologique à éliminer, détruire, ou inactiver.

Hormis éventuellement les alliages de métaux légers de type aluminium, dans le cas où l'on met en oeuvre des gels basiques ou acides, il n'existe aucune limitation quant au matériau qui constitue la surface (2) à décontaminer, en effet le gel selon l'invention permet de traiter sans aucun endommagement, toutes sortes de matériaux même fragiles.

Le gel selon l'invention ne génère aucune altération, érosion, attaque, chimique, mécanique ou physique du matériau traité. Le gel selon l'invention n'est donc en aucune manière préjudiciable à l'intégrité des matériaux traités et permet même leur réutilisation. Ainsi, des matériels sensibles tels que des équipements militaires sont préservés et pourront après leur décontamination être réutilisés, tandis que les monuments traités par le gel selon l'invention ne sont absolument pas dégradés et voient leur intégrité visuelle et structurale conservée.

Ce matériau du substrat (3) peut donc être choisi parmi par exemple les métaux comme l'acier inoxydable, les polymères tels que les matières plastiques ou caoutchoucs parmi lesquels on peut citer les PVC, PP, PE notamment HDPE, PMMA, PVDF, PC, les verres, les ciments, mortiers et bétons, les plâtres, les briques, la pierre naturelle ou artificielle, les céramiques.

Dans tous les cas (voir Exemple 9 et Figure 12), quel que soit le matériau, l'efficacité de décontamination par le gel selon l'invention est totale.

La surface traitée peut être peinte ou non peinte.

De manière particulièrement surprenante, il s'est avéré que le gel selon l'invention était particulièrement efficace sur les matériaux poreux tels que les matrices cimentaires comme les pâtes, les mortiers et les bétons, les briques, les plâtres, ou encore la pierre naturelle ou artificielle. En effet, la présence dans le gel selon l'invention d'un polymère super-absorbant permet une décontamination du matériau poreux sur une profondeur beaucoup plus importante qu'avec un gel équivalent sans polymère super-absorbant.

En d'autres termes, la présence d'un polymère super-absorbant dans le gel selon l'invention facilite la diffusion de l'agent actif de décontamination, par exemple de l'agent biocide dans la profondeur du matériau lorsqu'il s'agit de traiter des substrats poreux, notamment minéraux.

L'efficacité du traitement avec le gel selon l'invention est généralement totale, y compris sur les matériaux contaminés sur plusieurs millimètres de profondeur.

Il n'existe également aucune limitation quant à la forme, la géométrie et la taille de la surface à décontaminer, le gel selon l'invention et le procédé le mettant en oeuvre permettent le traitement de surfaces de grande taille, de géométries complexes, présentant par exemple des creux, angles, recoins.

Le gel selon l'invention assure le traitement efficace non seulement de surfaces horizontales telles que des planchers, mais aussi de surfaces verticales telles que des murs, ou de surfaces inclinées ou en surplomb telles que des plafonds.

Par rapport aux procédés de décontamination biologiques existants qui mettent en oeuvre des liquides tels que des solutions, le procédé de décontamination selon l'invention qui met en oeuvre un gel est particulièrement avantageux pour le traitement de matériaux de grande surface, non transportables et implantés à l'extérieur. En effet, le procédé selon l'invention du fait de la mise en oeuvre d'un gel, permet la décontamination *in situ* en évitant l'épandage de solutions chimiques dans l'environnement et la dispersion des espèces contaminantes.

Le gel selon l'invention peut être appliqué sur la surface à traiter par tous les procédés d'application connus de l'homme du métier.

Des procédés classiques sont la pulvérisation par exemple au pistolet ou l'application au moyen d'un pinceau, ou d'une taloche.

Pour l'application par pulvérisation du gel selon l'invention sur la surface à traiter, la solution colloïdale peut par exemple être véhiculée par l'intermédiaire d'une pompe basse pression, par exemple une pompe qui met en oeuvre une pression inférieure ou égale à 7 bar soit environ 7.10⁵ Pascals.

L'éclatement du jet de gel sur la surface peut être obtenu par exemple au moyen d'une buse à jet plat ou à jet rond.

La distance entre la pompe et la buse peut être quelconque, par exemple elle peut être de 1 à 50 m, notamment de 1 à 25 m.

Le temps de reprise de la viscosité suffisamment court des gels selon l'invention, permet aux gels pulvérisés d'adhérer à toutes les surfaces, par exemple à des parois.

La quantité de gel déposée sur la surface à traiter est généralement de 100 à 2000 g/m², de préférence de 500 à 1500 g/m², de préférence encore de 600 à 1000 g/m².

La quantité de gel déposée par unité de surface et, par voie de conséquence, l'épaisseur du gel déposé influence la vitesse de séchage.

Ainsi, lorsque l'on pulvérise un film, couche de gel d'une épaisseur de 0,5 mm à 2 mm sur la surface à traiter, le temps de contact efficace entre le gel et les matériaux est alors équivalent à son temps de séchage, période pendant laquelle le principe actif contenu dans le gel va interagir avec la contamination.

Dans le cas des substrats poreux, par exemple des matrices cimentaires, le temps d'action de la solution biocide ayant pénétré dans le coeur de matériau suite à l'action du polymère super-absorbant peut être supérieur au temps de séchage du gel, auquel cas il est généralement nécessaire soit de réaliser un remouillage avec la solution biocide, soit de répéter une pulvérisation du gel.

En outre, il a été montré de manière surprenante que la quantité de gel déposée lorsqu'elle se situe dans les plages mentionnées plus haut et en particulier lorsqu'elle est supérieure à 500 g/m² et notamment dans la plage de 500 à 1500 g/m², ce qui correspond à une épaisseur minimale de gel déposée par exemple supérieure à 500 µm pour une quantité de gel déposée supérieure à 500 g/m², permettait après séchage du gel d'obtenir une fracturation du gel sous la forme de paillettes millimétriques, par exemple d'une taille de 1 à 10 mm, de préférence de 2 à 5 mm aspirables.

La quantité de gel déposée et donc l'épaisseur de gel déposé, de préférence supérieure à 500 g/m² soit 500 µm, est le paramètre fondamental qui influence la taille des résidus secs formés après séchage du gel et qui assure ainsi que des résidus secs de taille millimétrique et non des résidus pulvérulents soient formés, de tels résidus étant facilement éliminés par un procédé mécanique et de préférence par aspiration.

Cependant, il est également à noter que grâce à l'agent tensio-actif à faible concentration, le séchage du gel est amélioré et conduit à un phénomène de fracturation homogène avec une taille des résidus secs mono dispersée et une aptitude accrue des résidus secs à se détacher du support.

Le gel est ensuite maintenu sur la surface à traiter pendant toute la durée nécessaire à son séchage. Au cours de cette étape de séchage dont on peut considérer qu'elle constitue la phase active du procédé selon l'invention, le solvant contenu dans le gel, à savoir généralement l'eau contenue dans le gel s'évapore jusqu'à l'obtention d'un résidu sec et solide.

La durée de séchage dépend de la composition du gel dans les gammes de concentration de ses constituants données plus haut, mais aussi, comme on l'a déjà précisé, de la quantité de gel déposée par unité de surface c'est-à-dire de l'épaisseur de gel déposé.

La durée de séchage dépend aussi des conditions climatiques à savoir de la température et de l'humidité relative de l'atmosphère dans laquelle se trouve la surface solide.

Le procédé selon l'invention peut être mis en oeuvre dans des conditions climatiques extrêmement larges, à savoir à une température T de 1°C à 50°C et à une humidité relative HR de 20% à 80%.

La durée de séchage du gel selon l'invention est donc généralement de 1 heure à 24 heures à une température T de 1°C à 50°C et à une humidité relative HR de 20% à 80%.

Il est à noter que la formulation du gel selon l'invention essentiellement du fait de la présence de tensio-actifs tels que les « Pluronics^{®} » assure généralement un temps de séchage qui est sensiblement équivalent au temps de contact (entre l'agent de décontamination, tel qu'un agent biocide, et les espèces biologiques notamment bio-toxiques à éliminer) qui est nécessaire, requis pour inactiver et/ou absorber les espèces contaminantes polluant le matériau. En d'autres termes, la formulation du gel assure un temps de séchage qui n'est autre que le temps d'inactivation des espèces contaminantes biologiques et qui est compatible avec la cinétique d'inhibition de la contamination biologique.

La surface spécifique de la charge minérale généralement utilisée qui est généralement de 50 m²/g à 300 m²/g, de préférence de 100 m²/g et la capacité d'absorption du gel selon l'invention permettent de piéger la contamination labile (surfacique) du matériau constituant la surface à traiter.

Le cas échéant, les espèces biologiques contaminantes sont inactivées dans la phase gélifiée. Après séchage du gel, la contamination inactivée est éliminée lors de la récupération du résidu de gel sec décrite plus bas.

A l'issue du séchage du gel, le gel se fracture de manière homogène pour donner des résidus secs solides millimétriques, par exemple d'une taille de 1 à 10 mm, de préférence de 2 à 5 mm non pulvérulents, généralement sous la forme de paillettes solides (5).

Les résidus secs peuvent contenir la ou les espèce (s) contaminante(s) inactivée(s) (6).

Les résidus secs, tels que des paillettes (5), obtenus à l'issue du séchage présentent une faible adhérence à la surface (2) du matériau décontaminé. De ce fait, les résidus secs obtenus après séchage du gel peuvent être facilement récupérés par simple brossage et/ou aspiration. Toutefois, les résidus secs peuvent aussi être évacués par jet de gaz, par exemple par jet d'air comprimé.

Ainsi, aucun rinçage n'est nécessaire et le procédé selon l'invention ne génère aucun effluent secondaire.

Le procédé selon l'invention réalise donc ainsi tout d'abord une importante économie de réactifs chimiques par rapport à un procédé de décontamination par lavage avec une solution. Ensuite du fait qu'un déchet sous la forme d'un résidu sec directement aspirable est obtenu, une opération de rinçage avec de l'eau ou avec un liquide est évitée. Il en résulte bien évidemment une diminution de la quantité d'effluents produits mais aussi une simplification notable en termes de filière de traitement et d'exutoire.

En raison de la composition majoritairement minérale du gel selon l'invention et de la faible quantité de déchets produits, le déchet sec peut être stocké ou dirigé vers une filière d'évacuation sans traitement préalable.

A titre d'exemple, dans le cas courant où l'on applique 1000 grammes de gel par m² de surface traitée, la masse de déchet sec produite est inférieure à 300 grammes par m².

Sur la Figure 2, on a illustré la décontamination par un gel non conforme à l'invention ne contenant pas de polymère super-absorbant d'un substrat poreux (21) contaminé par des spores en solution aqueuse (22). Le front de contamination (23) s'étend dans la profondeur du substrat (Figure 2A). Lorsque l'on applique un gel biocide (24) sur la surface (25) du substrat, le front de diffusion (26) de l'agent biocide s'étend peu dans la profondeur du substrat et reste en deçà du front de contamination (23) (Figure 2B). De ce fait, lorsque le gel est enlevé (Figure 2C) la zone assainie (27) s'étend peu en profondeur et il reste une contamination résiduelle (28) dans le substrat poreux (21).

Sur la Figure 3, on a illustré la décontamination, par un gel conforme à l'invention contenant un polymère super-absorbant, d'un substrat poreux (31) contaminé par des spores en solution aqueuse (32). Le front de contamination (33) s'étend dans la profondeur du substrat (Figure 3A). Lorsque l'on applique un gel biocide contenant le super-absorbant(34) sur la surface (35) du substrat, le front de diffusion (36) de l'agent biocide s'étend dans la profondeur du substrat et va au-delà du front de contamination (Figure 3B). De ce fait, la zone assainie (37) s'étend en profondeur (P) et il ne reste plus de contamination résiduelle dans le substrat poreux.

L'invention va maintenant être décrite en référence aux exemples suivants, donnés à titre illustratif et non limitatif.

### EXEMPLES :

### Exemple 1 :

Dans cet exemple, on étudie la cinétique d'inhibition des spores de Bacillus thuringiensis, dans différentes solutions biocides liquides contenant différents agents actifs de décontamination à diverses concentrations, à savoir : NaOCl à 4,8%, NaOH à 1M ; HNO₃ à 0,5M, CHP (chlorure d'hexadecyl pyridinium) à 2%. Des solutions comparatives contenant le tensio-actif Pluronic^{®} P 8020 à 1%, ou le tensio-actif KR8 (alcool gras éthoxylé) à 1% ont également été utilisées.

### Protocole expérimental :

L'expérimentation consiste à mettre en contact, sous agitation, 2x10⁶ spores avec 1 ml de solution biocide liquide.

A l'issue de 1 heure et 24 heures d'agitation, des prélèvements sont effectués pour révéler l'activité biologique du mélange. La révélation consiste alors à déposer une goutte de mélange sur un milieu nutritif (Gel Agar) et à dénombrer, à l'issue d'une période d'incubation de 16 heures à 30°C, le nombre de colonies formées. Chacune des colonies étant le résultat d'une spore inactivée.

Les résultats des essais sont donnés sur la Figure 4 où le nombre de spores résiduelles est donné pour chacune des solutions biocides et des solutions comparatives à des temps de contact de 1 heure et 24 heures.

La Figure 4 montre notamment que le Pluronic^{®} P 8020 et le tensio-actif KR8 n'ont pas d'action sur les spores.

### Exemple 2 :

Dans cet exemple, on étudie la cinétique d'inhibition des spores de Bacillus thuringiensis, dans différentes solutions biocides liquides contenant différentes bases à diverses concentrations, à savoir : NaOH à 0,5 M, NaOH à 1M ; NaOH à 5M, KOH à 0,5M, KOH à 1M, et KOH à 5M.

Le protocole expérimental utilisé est analogue à celui décrit plus haut dans l'exemple 1. Seul le nombre de prélèvements de mélange est augmenté (1 heure, 2 heures, 3 heures, 4 heures, 5 heures) de manière à déterminer la cinétique d'inhibition des spores dans le milieu biocide considéré.

Les résultats des essais sont donnés sur la Figure 5 où le nombre de spores résiduelles est donné pour chacune des solutions biocides à des temps de contact de 1 heure, 2 heures, 3 heures, 4 heures, 5 heures.

La Figure 5 montre que l'augmentation de la concentration en agent biocide permet d'accroître considérablement les vitesses d'inhibition des spores de Bacillus thuringiensis.

### Exemple 3 :

Dans cet exemple, on étudie l'influence de la concentration en hydroxyde de sodium dans un gel de la présente invention sur la durée de séchage.

Le gel a la composition suivante en pourcentages massiques :
- Alumine : 14%
- Solution d'hydroxyde de sodium (concentration variable) : 85%
- Tensio-actif (Pluronic^{®} P8020) : 0,7%
- Polymère super-absorbant : Polyacrylate de sodium Norsocryl^{®} S35 : 0,3%.

### Protocole expérimental :

Les gels, de concentration en hydroxyde de sodium variable (0 M, 1 M, 5 M et 10 M) sont étalés sur un support métallique inerte sur une épaisseur contrôlée de 1 mm. Le support métallique contenant le film de gel est alors placé dans une enceinte climatique équipée d'une balance de précision qui assure le suivi de la perte de masse du gel au cours du temps. L'enceinte climatique est régulée à une température de 22°C et à une humidité relative de 60%.

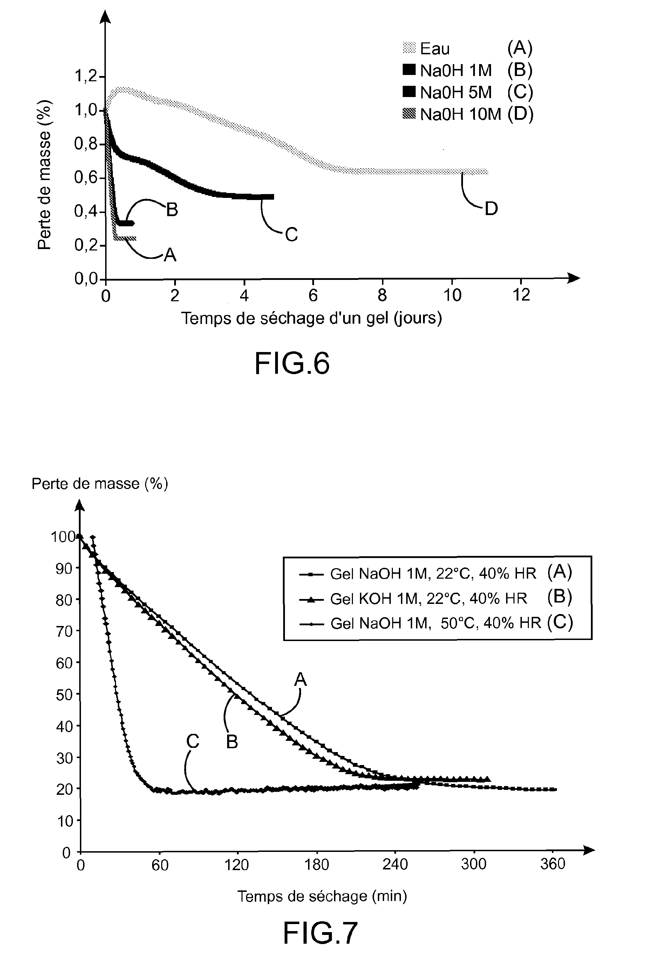

Les courbes de la Figure 6 montrent que le caractère hygroscopique de l'hydroxyde de sodium (mais aussi de l'hydroxyde de potassium) ralentit le phénomène de séchage du gel. De ce fait, le temps de contact entre l'agent de décontamination, à savoir la solution biocide, et la contamination biologique se trouve considérablement augmenté.

### Exemple 4 :

Dans cet exemple, on étudie l'influence de la température sur la cinétique de séchage d'un gel à base de NAOH 1M ; et la cinétique de séchage d'un gel à base de KOH 1M.

Les gels ont la composition suivante en pourcentages massiques :
- Alumine : 14%
- Solution d'hydroxyde de sodium (1M) : 85%
- Tensio-actif (Pluronic^{®} P8020) : 0,7%
- Polymère super-absorbant : Polyacrylate de sodium Norsocryl^{®} S35 : 0,3%.
Ou bien,
- Alumine : 14%
- Solution d'hydroxyde de potassium (1M) : 85%
- Tensio-actif (Pluronic^{®} P8020) : 0,7%
- Polymère super-absorbant : Polyacrylate de sodium Norsocryl^{®} S35 : 0,3%.
Le protocole expérimental utilisé est analogue à celui décrit plus haut dans l'exemple 3. L'enceinte climatique est dans un cas régulée à une température de 22°C et 40% d'humidité relative (gel NaOH 1 M, gel KOH 1 M), dans un autre à une température de 50°C et 40% d'humidité relative (gel NaOH 1 M, courbe de gauche C).

Les courbes de la Figure 7 montrent que le temps de séchage du gel à base de NaOH 1M à 22°C est légèrement plus long que celui du gel à base de KOH 1M à la même température tandis que le temps de séchage du gel de NaOH 1M à 50°C est fortement réduit.

### Exemple 5 :

Dans cet exemple, on étudie l'influence de l'épaisseur de gel déposé sur la cinétique de séchage d'un gel de la présente invention à base de NaOH 1M.

Le gel a la composition suivante en pourcentages massiques :
- Alumine : 14%
- Solution d'hydroxyde de sodium (1M) : 85%
- Tensio-actif (Pluronic^{®} P8020) : 0,7%
- Polymère super-absorbant : Polyacrylate de sodium Norsocryl^{®} S35 : 0,3%.

Le protocole expérimental utilisé est analogue à celui décrit plus haut dans l'exemple 3. L'enceinte climatique est dans ce cas régulée à une température de 22°C et 40% d'humidité relative. Seule l'épaisseur de gel déposé sur le support métallique varie de 1 mm à 2 mm.

Les courbes de la Figure 8 montrent que le temps de séchage est nettement allongé lorsque l'on passe d'une épaisseur de gel déposé de 1 mm (courbe A) à une épaisseur de gel déposé de 2 mm (courbe B).

### Exemple 6 :

Dans cet exemple, on étudie l'influence du polymère super-absorbant sur l'efficacité de la décontamination biologique d'un mortier exprimée par le nombre de spores de Bacillus thuringiensis sur un échantillon de mortier.

### Protocole expérimental :

Les échantillons de mortier sont contaminés par dépôt d'une goutte d'eau d'un volume de 100 µl contenant 2x10⁷ spores de Bacillus thuringiensis.

Après diffusion de la solution contaminante dans la profondeur du matériau cimentaire, les gels biocides de décontamination sont étalés sur la face contaminée des échantillons de mortier. La quantité de gel déposé est égale à 1000 g/m².

A l'issue de 24 heures de séchage, les paillettes de gel sec formées sont éliminées de l'échantillon de mortier. Ce dernier est alors immergé dans une solution nutritive Luria Broth maintenue sous agitation pendant 3 heures à une température de 37°C.

La révélation de l'activité biologique résiduelle des échantillons de mortier consiste alors à prélever un volume connu de solution nutritive Luria Broth dans lequel ont trempé les échantillons de mortier et à le déposer sur un gel agarose. Après 24 heures d'incubation, le dénombrement des colonies de bactéries permet de révéler le nombre de spores non inactivées par le gel biocide de décontamination.

Le gel comprenant un polymère super-absorbant a la composition suivante en pourcentages massiques :
- Alumine : 14%
- Solution d'hydroxyde de sodium (1M) : 85%
- Tensio-actif (Pluronic^{®} P8020) : 0,7%
- Polymère super-absorbant : Polyacrylate de sodium Norsocryl^{®} S35 : 0,3%

Le gel exempt de polymère absorbant à la même composition sauf que le polymère super-absorbant est omis.

Le graphique de la Figure 9 montre que l'ajout de polymère super-absorbant permet d'accroître significativement l'efficacité de la décontamination d'un matériau poreux tel qu'un mortier qui est contaminé en profondeur sur une épaisseur de plusieurs millimètres.

### Exemple 7 :

Dans cet exemple, on étudie l'influence de la concentration en tensio-actif, à savoir le Pluronic^{®} P 8020, sur le pouvoir d'adhésion des paillettes de gel sec.

### Protocole expérimental :

Le gel est appliqué sur une lame d'acier inoxydable flexible (feuille calibrée de chez Outillage francilien), dont les propriétés mécaniques sont connues (25 µm d'épaisseur, longueur 2 cm, largeur 1 cm et un module de Young de 2.10¹¹ Pa), l'une des extrémités est fixe et l'autre libre. On nivelle la surface de la couche de gel avec un racleur approprié afin d'y déposer une épaisseur constante de un mm.

On ajoute deux caméras, une caméra placée au-dessus de la couche de gel permet de visualiser l'apparition des fractures et une autre placée sur le côté permet de mesurer l'évolution de l'épaisseur de la couche de gel au cours du temps. L'adhésion des paillettes est étudiée par analyses des images obtenues par les deux caméras.

Le gel étudié est le gel exempt de polymère super-absorbant dans lequel on fait varier la concentration en tensio-actif. Les autres composés étant maintenus à des teneurs massiques égales à celles de l'exemple 6.

Les concentrations en tensio-actifs sont de 0,10 g/L, et 50 g/L.

Le graphique de la Figure 10 montre que dans la gamme de concentration qui nous intéresse (< 10 g/l), l'augmentation de la concentration en Pluronic^{®} engendre une diminution de l'adhésion des paillettes. La récupération du déchet de gel sec par brossage et/ou aspiration s'en retrouve alors facilitée.

### Exemple 8 :

Dans cet exemple, on étudie l'influence de la concentration en tensio-actif, à savoir le Pluronic^{®} P 8020, sur le nombre de paillettes de gel sec formées.

Le gel étudié est le gel de l'exemple 7 dans lequel on fait varier la concentration en tensio-actif.

Les concentrations en tensio-actifs sont de 0,10 g/L, et 50 g/L.

Le protocole expérimental utilisé est rigoureusement le même que celui utilisé dans l'exemple 7.

Le graphique de la Figure 11 montre que l'ajout de Pluronic^{®} à la formulation du gel engendre une diminution du nombre de paillettes. L'ajout de Pluronic^{®} permet d'améliorer la ténacité de la matrice gélifiée face à la fracturation induite par le séchage : le gel va se fracturer plus difficilement, le nombre de fractures sera moindre et le nombre de paillettes sera ainsi plus petit. En ce qui concerne le procédé de décontamination, la diminution du nombre de paillettes est bénéfique : les paillettes étant plus grosses, le déchet sera non pulvérulent lors de la phase de récupération du déchet par brossage et/ou aspiration.

### Exemple 9 :

Dans cet exemple, on étudie l'efficacité du gel de traitement selon l'invention sur une contamination par des spores de Bacillus Thuringiensis en fonction de la nature du matériau traité.

Le gel étudié est le gel comprenant un polymère absorbant de l'exemple 6.

Le protocole expérimental est identique à celui de l'exemple 6. Les matériaux étudiés étant des matériaux non poreux, ces derniers sont traités par le gel après une phase d'évaporation de la goutte contaminante de 30 minutes. Cette phase d'évaporation correspondant au souhait de traiter une contamination sèche, a priori la plus préjudiciable pour le procédé de l'invention, recouvrant la surface des matériaux.

Le graphique de la Figure 12 montre qu'après récupération du gel, quel que soit le matériau traité (Acier inoxydable, acier peint, Verre, PVC, PP, PMMA, HDPE, PVDF, PC), la décontamination est totale sans que le matériau ne soit altéré.

Cet exemple montre l'efficacité et la polyvalence du gel selon l'invention.

### RÉFÉRENCES

[1] JENEVEIN. E, *"Cleaning composition for neutralizing biological and chemical weapons removal agents",* US-B2-7,026,274.
[2] SCHILLING. A, HODGE. R *"Peracid-based large* area *decontamination",* Patent n° US-A1- 2006/0073067.
[3] CONERLY. L, EHNTHOLT. D, LOUIE. A, WHELAN. R *"Chemical and*/*or biological decontamination system",* US-A1-2003/0109017.
[4] TUCKER. M, COMSTOCK. R *"Decontamination formulation with sorbent additive",* US-A1- 2004/0022867.
[5] ROGERS. J.V, SABOURIN. C.L.K, CHOI. Y.W "Decontamination assessment of bacillus subtilis, and Geobacillus stearothermophilus spores on indoor surfaces using a hydrogen peroxide gas generator", 2005.
[6] JOSSE. D, BOUDRY. I, NAUD. N "Décontamination cutanée vis-à-vis des agents organophosphorés et de l'ypérite au soufre : Bilan et perspectives", Médecine et armées, vol. 34, n°1, pages 33-36, 2006.
[7] HOFFMAN. D, Mc GUIRE. R *"Oxidizer gels for detoxification of chemical and biological agents",* US-B1- 6,455,751.
[8] HARPER. B, LARSEN. L "A comparison of decontamination technologies for biological agents on selected commercial surface materials", Biological weapons improved response program, April 2001.
[9] FAURE. S, FOURNEL. B, FUENTES. P, LALLOT. Y. *"Procédé de traitement d'une surface par un gel de traitement, et gel de traitement",* FR-A1-2 827 530.
[10] FAURE. S, FUENTES. P, LALLOT. Y. *"Gel aspirable pour la décontamination* de *surfaces* et *utilisation",* FR-A1-2 891 470.

## Revendications

1. Gel de décontamination biologique, constitué par une solution colloïdale comprenant :
- 5 à 30% en masse, de préférence 5 à 25% en masse, de préférence encore 8 à 20% en masse par rapport à la masse du gel, d'au moins un agent viscosant inorganique ;
- 0,5 à 10 mol/L de gel, de préférence 1 à 10 mol/L de gel, d'au moins un agent actif de décontamination biologique ;
- 0,05 à 5% en masse, de préférence 0,05 à 2% en masse par rapport à la masse du gel, d'au moins un polymère super-absorbant;
- 0,1 à 2% en masse par rapport à la masse du gel, d'au moins un agent tensio-actif ;
- et le reste de solvant.

2. Gel selon la revendication 1, dans lequel l'agent viscosant inorganique est choisi parmi les alumines, les silices, les aluminosilicates, les argiles, et leurs mélanges ; de préférence l'agent viscosant inorganique est choisi parmi les silices pyrogénées, les silices précipitées, les silices hydrophiles, les silices hydrophobes, les silices acides, les silices basiques, et leurs mélanges ; de préférence encore l'agent viscosant inorganique est constitué par un mélange d'une silice précipitée et d'une silice pyrogénée

3. Gel selon la revendication 2, dans lequel l'agent viscosant inorganique est constitué par une ou plusieurs alumine(s) représentant de 5% à 30% en masse, de préférence de 8 à 17% en masse par rapport à la masse du gel.

4. Gel selon l'une quelconque des revendications précédentes, dans lequel l'agent actif de décontamination biologique est choisi parmi les bases telles que l'hydroxyde de sodium, l'hydroxyde de potassium, et leurs mélanges ; les acides tels que l'acide nitrique, l'acide phosphorique, l'acide chlorhydrique, l'acide sulfurique, et leurs mélanges; les agents oxydants tels que les peroxydes, permanganates, persulfates, l'ozone, les hypochlorites, et leurs mélanges; les sels d'ammonium quaternaires tels que les sels d'hexacétylpyridinium ; et leurs mélanges.

5. Gel selon l'une quelconque des revendications précédentes, dans lequel le polymère super-absorbant est choisi parmi les poly(méth)acrylates de sodium, les amidons greffés par un polymère (méth)acrylique, les amidons hydrolysés greffés par un polymère (méth)acrylique ; les polymères à base d'amidon, de gomme, et de dérivé celiulosique ; et leurs mélanges.

6. Gel selon l'une quelconque des revendications précédentes, dans lequel l'agent tensio-actif est choisi parmi les agents tensio-actifs non ioniques tels que les copolymères blocs, séquencés comme les copolymères séquencés d'oxyde d'éthylène et d'oxyde de propylène, et les acides gras éthoxylés ; et leurs mélanges.

7. Gel selon l'une quelconque des revendications précédentes, dans lequel le solvant est choisi parmi l'eau, les solvants organiques et leurs mélanges.

8. Procédé de décontamination biologique d'une surface d'un substrat solide contaminée par au moins une espèce biologique se trouvant sur ladite surface et éventuellement sous ladite surface dans la profondeur du substrat, dans lequel on réalise au moins un cycle comprenant les étapes successives suivantes :
a) on applique le gel selon l'une quelconque des revendications 1 à 7 sur ladite surface ;
b) on maintient le gel sur la surface au moins pendant une durée suffisante pour que le gel détruise et/ou inactive et/ou absorbe l'espèce biologique, et pour que le gel sèche et forme un résidu sec et solide contenant ladite espèce biologique ;
c) on élimine le résidu sec et solide contenant ladite espèce biologique.

9. Procédé selon la revendication 8, dans lequel le substrat solide est un substrat poreux, de préférence un substrat minéral poreux.

10. Procédé selon la revendication 8 ou 9, dans lequel le substrat est au moins en un matériau choisi parmi les métaux comme l'acier inoxydable ; les polymères tels que les matières plastiques ou caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments ; les mortiers et bétons ; les plâtres ; les briques ; la pierre naturelle ou artificielle ; les céramiques.

11. Procédé selon l'une quelconque des revendications 8 à 10, dans lequel l'espèce biologique est choisie parmi les bactéries, les champignons, les levures, les virus, les toxines, les spores et les protozoaires ; de préférence l'espèce biologique est choisie parmi les espèces bio-toxiques telles que les spores pathogènes comme par exemple les spores de Bacillus anthracis, les toxines comme par exemple la toxine botulique, et les virus.

12. Procédé selon l'une quelconque des revendications 8 à 11, dans lequel le gel est appliqué sur la surface à raison de 100 g à 2000 g de gel par m² de surface, de préférence de 500 g à 1500 g de gel par m², de préférence encore de 600 g à 1000 g de gel par m² de surface.

13. Procédé selon l'une quelconque des revendications 8 à 12, dans lequel le gel est appliqué sur la surface solide par pulvérisation, au pinceau ou avec une taloche.

14. Procédé selon l'une quelconque des revendications 8 à 13, dans lequel lors de l'étape b), le séchage est réalisé à une température de 1°C à 50°C, de préférence de 15°C à 25°C, et sous une humidité relative de 20% à 80%, de préférence de 20% à 70%.

15. Procédé selon l'une quelconque des revendications 8 à 14, dans lequel le gel est maintenu sur la surface pendant une durée de 2 à 72 heures, de préférence de 2 à 48 heures, de préférence encore de 5 à 24 heures.

16. Procédé selon l'une quelconque des revendications 8 à 15, dans lequel le résidu sec et solide se présente sous la forme de particules, par exemple de paillettes, d'une taille de 1 à 10 mm, de préférence de 2 à 5 mm.

17. Procédé selon l'une quelconque des revendications 8 à 16, dans lequel le résidu sec et solide est éliminé de la surface solide par brossage et/ou aspiration.

18. Procédé selon l'une quelconque des revendications 8 à 17, dans lequel le cycle décrit est répété de 1 à 10 fois en utilisant le même gel lors de tous les cycles ou en utilisant des gels différents lors d'un ou de plusieurs cycle(s).

19. Procédé selon l'une quelconque des revendications 8 à 18, dans lequel, lors de l'étape b), le gel, avant séchage total, est remouillé avec une solution d'un agent de décontamination biologique, de préférence avec la solution de l'agent actif biologique du gel appliqué lors de l'étape a) dans le solvant de ce gel.

## Patentansprüche

1. Biologisches Dekontaminationsgel, das sich aus einer Kolloidlösung zusammensetzt, die Folgendes umfasst:
- 5 bis 30 Massen-%, vorzugsweise 5 bis 25 Massen-%, bevorzugter 8 bis 20 Massen-% bezogen auf die Masse des Gels wenigstens eines anorganischen, Viskosität verleihenden Mittels,
- 0,5 bis 10 Mol/l Gel, vorzugsweise 1 bis 10 Mol/l Gel wenigstens eines biologischen Dekontaminationswirkstoffs,
- 0,05 bis 5 Massen-%, vorzugsweise 0,05 bis 2 Massen-% bezogen auf die Masse des Gels wenigstens eines superabsorbierenden Polymers,
- 0,1 bis 2 Massen-% bezogen auf die Masse des Gels wenigstens eines Tensids
- und der Rest Lösungsmittel.

2. Gel gemäß Anspruch 1, bei dem das anorganische, Viskosität verleihende Mittel aus Aluminiumoxiden, Siliciumdioxiden, Aluminiumsilikaten, Tonen, und ihren Gemischen ausgewählt ist; das anorganische, Viskosität verleihende Mittel vorzugsweise aus pyrogenen Siliciumdioxiden, gefällten Siliciumdioxiden, hydrophilen Siliciumdioxiden, hydrophoben Siliciumdioxiden, sauren Siliciumdioxiden, basischen Siliciumdioxiden, und ihren Gemischen ausgewählt ist; und sich das anorganische, Viskosität verleihende Mittel bevorzugter aus einem Gemisch aus einem gefällten Siliciumdioxid und einem pyrogenen Siliciumdioxid zusammensetzt.

3. Gel gemäß Anspruch 2, bei dem sich das anorganische, Viskosität verleihende Mittel aus einem oder mehreren Aluminiumoxid(en) zusammensetzt, die 5 bis 30 Massen-%, vorzugsweise 8 bis 17 Massen-% bezogen auf die Masse des Gels darstellen.

4. Gel gemäß einem der vorangehenden Ansprüche, bei dem der biologische Dekontaminationswirkstoff aus Basen wie etwa Natriumhydroxid, Kaliumhydroxid, und ihren Gemischen; Säuren wie etwa Salpetersäure, Phosphorsäure, Salzsäure, Schwefelsäure und ihren Gemischen; Oxidationsmitteln wie etwa Peroxiden, Permanganaten, Persulfaten, Ozon, Hypochloriten und ihren Gemischen; quaternären Ammoniumsalzen wie etwa Hexacetylpyridiniumsalzen; und ihren Gemischen ausgewählt ist.

5. Gel gemäß einem der vorangehenden Ansprüche, bei dem das superabsorbierende Polymer aus Natriumpoly(meth)acrylaten, auf ein (Meth)acrylpolymer gepfropften Amidonen, durch ein (Meth)acrylpolymer gepfropften, hydrolysierten Amidonen; Polymeren auf der Basis von Amidon, Gummi, und Cellulosederivaten; und ihren Gemischen ausgewählt ist.

6. Gel gemäß einem der vorangehenden Ansprüche, bei dem das Tensid aus nichtionischen Tensiden wie etwa Blockcopolymeren, wie Ethylenoxid- und Propylenoxid-Blockcopolymeren, und ethoxylierten Fettsäuren; und ihren Gemischen ausgewählt ist.

7. Gel gemäß einem der vorangehenden Ansprüche, bei dem das Lösungsmittel aus Wasser, organischen Lösungsmitteln und ihren Gemischen ausgewählt ist.

8. Verfahren zur biologischen Dekontamination einer Oberfläche eines durch wenigstens eine biologische Spezies kontaminierten, sich auf dieser Oberfläche und gegebenenfalls in der Tiefe des Substrats auf dieser Oberfläche befindlichen, festen Substrats, bei dem wenigstens eine Abfolge ausgeführt wird, die die folgenden, aufeinander folgenden Schritte umfasst:
a) das Gel gemäß einem der Ansprüche 1 bis 7 wird auf diese Oberfläche aufgebracht,
b) das Gel wird mindestens während einer Dauer auf der Oberfläche belassen, in der das Gel die biologische Spezies zerstört und/oder inaktiviert und/oder absorbiert und in der das Gel trocknet und einen trockenen und festen, die besagte biologische Spezies enthaltenden Rückstand bildet, und
c) der die besagte biologische Spezies enthaltende trockene und feste Rückstand entfernt wird.

9. Verfahren gemäß Anspruch 8, bei dem das feste Substrat ein poröses Substrat, vorzugsweise ein mineralisches poröses Substrat ist.

10. Verfahren gemäß Anspruch 8 oder 9, bei dem das Substrat wenigstens aus einem Material ist, das aus Metallen wie Edelstahl; Polymeren wie etwa Kunststoffmaterialien oder Kautschuken wie Poly(vinylchlorid)en oder PVC, Polypropylenen oder PP, Polyethylenen oder PE, insbesondere Polyethylenen hoher Dichte oder HDPE, Poly(methylmethacrylat)en oder PMMA, Poly(vinylidenfluorid)en oder PVDF, Polycarbonaten oder PC; Gläsern; Zementen; Mörteln und Betonen; Gipsen; Ziegeln;
natürlichen oder künstlichen Steinen; und keramischen Materialien ausgewählt ist.

11. Verfahren gemäß einem der Ansprüche 8 bis 10, bei dem die biologische Spezies aus Bakterien, Pilzen, Hefen, Viren, Toxinen, Sporen und Protozoen ausgewählt ist; und die biologische Spezies vorzugsweise aus biotoxischen Spezies wie etwa pathogenen Sporen wie zum Beispiel Sporen von Bacillus anthracis, Toxinen wie zum Beispiel dem Botulinustoxin und Viren ausgewählt ist.

12. Verfahren gemäß einem der Ansprüche 8 bis 11, bei dem das Gel auf die Oberfläche in einer Menge von 100 g bis 2000 g je m² Oberfläche, vorzugsweise 500 g bis 1500 g Gel je m², bevorzugter 600 g bis 1000 g Gel je m² Oberfläche aufgebracht wird.

13. Verfahren gemäß einem der Ansprüche 8 bis 12, bei dem das Gel durch Pulverisierung, mittels eines Pinsels oder mit einem Reibebrett aufgebracht wird.

14. Verfahren gemäß einem der Ansprüche 8 bis 13, bei dem während des Schritts b) die Trocknung bei einer Temperatur von 1 °C bis 50 °C, vorzugsweise 15 °C bis 25 °C, und bei einer relativen Feuchtigkeit von 20 % bis 80 %, vorzugsweise 20 % bis 70 %, durchgeführt wird.

15. Verfahren gemäß einem der Ansprüche 8 bis 14, bei dem das Gel während einer Dauer von 2 bis 72 Stunden, vorzugsweise 2 bis 48 Stunden und bevorzugter 5 bis 24 Stunden auf der Oberfläche belassen wird.

16. Verfahren gemäß einem der Ansprüche 8 bis 15, bei dem der trockene und feste Rückstand die Form von Teilchen, zum Beispiel Plättchen mit einer Größe von 1 bis 10 mm, vorzugsweise 2 bis 5 mm, aufweist.

17. Verfahren gemäß einem der Ansprüche 8 bis 16, bei dem der trockene und feste Rückstand durch Bürsten und/oder Absaugen von der Oberfläche entfernt wird.

18. Verfahren gemäß einem der Ansprüche 8 bis 17, bei dem die beschriebene Abfolge 1 bis 10 Mal wiederholt wird, indem dasselbe Gel während aller Abfolgen verwendet wird oder indem verschiedene Gele während einer oder mehrerer Abfolge(n) verwendet werden.

19. Verfahren gemäß einem der Ansprüche 8 bis 18, bei dem während des Schritts b) das Gel vor der vollständigen Trocknung wieder mit einer Lösung eines biologischen Dekontaminierungsmittels, vorzugsweise mit der Lösung des biologischen Wirkstoffs des Gels befeuchtet wird, der während des Schritts a) in dem Lösungsmittel dieses Gels angewandt wird.

## Claims

1. A biological decontamination gel, consisting of a colloidal solution comprising:
- from 5 to 30% by mass, preferably 5 to 25% by mass, still preferably 8 to 20% by mass based on the mass of the gel, of at least one inorganic viscosifying agent;
- from 0.5 to 10 mol/L of gel, preferably 1 to 10 mol/L of gel, of at least one active biological decontamination agent;
- 0.05 to 5% by mass, preferably 0.05 to 2% by mass, based on the mass of the gel, of at least one super-absorbent polymer;
- 0.1 to 2% by mass, based on the mass of the gel, of at least one surfactant;
- and the remainder of solvent.

2. The gel according to claim 1, wherein the inorganic viscosifying agent is selected from aluminas, silicas, aluminosilicates, clays and mixtures thereof; preferably the inorganic viscosifying agent is selected from pyrogenated silicas, precipitated silicas, hydrophilic silicas, hydrophobic silicas, acid silicas, basic silicas, and mixtures thereof; more preferably the inorganic viscosifying agent consists of a mixture of a precipitated silica and a pyrogenated silica.

3. The gel according to claim 2, wherein the inorganic viscosifying agent consists in one or more alumina(s) representing from 5% to 30% by mass, preferably from 8 to 17% by mass, based on the mass of the gel.

4. The gel according to any one of the preceding claims, wherein the active biological decontamination agent is selected from bases such as sodium hydroxide, potassium hydroxide, and mixtures thereof; acids such as nitric acid, phosphoric acid, hydrochloric acid, sulfuric acid, and mixtures thereof; oxidizing agents such as peroxides, permanganates, persulfates, ozone, hypochlorites, and mixtures thereof; quaternary ammonium salts such as hexacetylpyridinium salts; and mixtures thereof.

5. The gel according to any one of the preceding claims, wherein the super-absorbent polymer is selected from sodium poly(meth)acrylates, starches grafted with a (meth)acrylic polymer, hydrolyzed starches grafted with a (meth)acrylic polymer; polymers based on starch, on gum, and on a cellulose derivative; and mixtures thereof.

6. The gel according to any one of the preceding claims, wherein the surfactant is selected from non-ionic surfactants such as block copolymers like block copolymers of ethylene oxide and of propylene oxide, and ethoxylated fatty acids; and mixtures thereof.

7. The gel according to any one of the preceding claims, wherein the solvent is selected from water, organic solvents and mixtures thereof.

8. A method for biological decontamination of a surface of a solid substrate contaminated by at least one biological species found on said surface and possibly under said surface in the depth of the substrate, wherein at least one cycle is carried out, comprising the following successive steps:
a) the gel according to any one of claims 1 to 7 is applied on said surface;
b) the gel is maintained on the surface at least for a sufficient time so that the gel destroys and/or inactivates and/or absorbs the biological species, and so that the gel dries and forms a dry and solid residue containing said biological species;
c) the dry and solid residue containing said biological species is removed.

9. The method according to claim 8, wherein the solid substrate is a porous substrate, preferably a porous mineral substrate.

10. The method according to claim 8 or 9, wherein the substrate is made of at least one material selected from metals such as stainless steel; polymers such as plastic materials or rubbers like poly(vinyl chloride)s or PVC, polypropylenes or PP, polyethylenes or PE notably high density polyethylenes or HDPE, poly(methyl methacrylate)s or PMMA, poly(vinylidene fluoride)s or PVDF, polycarbonates or PC; glasses, cements; mortars and concretes; plasters; bricks; natural or artificial stone; ceramics.

11. The method according to any one of claims 8 to 10, wherein the biological species is selected from bacteria, fungi, yeasts, viruses, toxins, spores and protozoans; preferably the biological species is selected from bio-toxic species such as pathogenic spores, such as for example the spores of *Bacillus anthracis,* toxins such as for example *Botulinum* toxin, and viruses.

12. The method according to any one of claims 8 to 11, wherein the gel is applied on the surface in an amount from 100 g to 2,000 g of gel per m² of surface, preferably from 500 to 1,500 g of gel per m², still preferably from 600 g to 1,000 g of gel per m² of surface.

13. The method according to any one of claims 8 to 12, wherein the gel is applied on the solid surface by spraying, with a brush or with a hawk.

14. The method according to any one of claims 8 to 13, wherein, during step b), the drying is carried out at a temperature from 1°C to 50°C, preferably from 15°C to 25°C, and under a relative humidity from 20% to 80%, preferably from 20% to 70%.

15. The method according to any one of claims 8 to 14, wherein the gel is maintained on the surface for a duration from 2 to 72 hours, preferably from 2 to 48 hours, still preferably from 5 to 24 hours.

16. The method according to any one of claims 8 to 15, wherein the dry and solid residue appears as particles, for example flakes, with a size from 1 to 10 mm, preferably from 2 to 5 mm.

17. The method according to any one of claims 8 to 16, wherein the dry and solid residue is removed from the solid surface by brushing and/or suction.

18. The method according to any one of claims 8 to 17, wherein the described cycle is repeated from 1 to 10 times by using the same gel during all the cycles or by using different gels during one or more cycle(s).

19. The method according to any one of claims 8 to 18, wherein, during step b), the gel, before total drying, is re-wetted with a solution of a biological decontamination agent, preferably with the solution of the active biological agent of the gel applied during step a) in the solvent of this gel.
